# EUROPÄISCHE PATENTSCHRIFT

(11) **EP 0 841 314 B1**
(45) Veröffentlichungstag und Bekanntmachung des Hinweises auf die Patenterteilung: **26.02.2003**
(21) Anmeldenummer: 97119671.2
(22) Anmeldetag: 11.11.1997
(51) Int. Cl.: C07B 35/02, C07C 29/17, C07C 33/03, C07C 49/04, C07C 45/62

(54) **Katalytische Hydrierung unter Verwendung von amorphen Metalllegierungen**
Catalytic hydrogenation using amorphous metal alloys
Hydrogénation catalytique utilisant les alliages métalliques amorphes

(30) Priorität: 11.11.1996 EP 96118038
(43) Veröffentlichungstag der Anmeldung: 13.05.1998
(73) Patentinhaber: F. HOFFMANN-LA ROCHE AG, 4070 Basel (CH)
(72) Erfinder: Jansen, Michael, 68870 Bartenheim (FR); Rehren, Claus, 63739 Aschaffenburg (DE)
(74) Vertreter: Kellenberger, Marcus, Dr.

(56) Entgegenhaltungen:
- EP-A- 0 173 088
- WO-A-94/06738
- WO-A-96/01304
- WO-A-97/38955
- DE-A- 4 405 029
- CHEMICAL ABSTRACTS, vol. 105, no. 25, 22.Dezember 1986 Columbus, Ohio, US; abstract no. 227049h, T. O. OMARKULOV: "Study of the kinetics and selectivity of the hydration of the C20 acetylene carbinol (dehydroisophytol) in a stream on supported palladium catalysts under hydrogen pressure." Seite 834; Spalte 1; XP002057007 & ZH. PRIKL. KHIM. (LENINGRAD), Bd. 58, Nr. 7, 1985, Seiten 1536-1539,
- A. MOLNAR: "Selective hydrogenation of alkynes over metallic glasses" JOURNAL OF CATALYSIS, Bd. 101, 1986, MN US, Seiten 67-72, XP002057006

## Beschreibung

Die vorliegende Erfindung betrifft ein Verfahren zur katalytischen Hydrierung von organischen Verbindungen an einem Katalysator aus amorphen Metall-Legierungen unter nahekritischen oder überkritischen Bedingungen des Lösungsmittels.

Amorphe Metall-Legierungen werden in der Technik auch als metallische Gläser bezeichnet. Sie können zum Beispiel durch Schockkühlung der Legierungsschmelzen auf einem gekühlten, rotierenden Kupferrad hergestellt werden. Abhängig von der Oberflächenbeschaffenheit des Kupferrades und den konkreten Verfahrensbedingungen werden dünne Metallbänder (bei glatter Kupferoberfläche) oder flockenartige Partikel, sogenannte Flakes, (bei rauher Kupferoberfläche) erhalten. Die Dicke der Metallbänder beziehungsweise der Flakes liegt im Bereich zwischen 5 und 50 µm, vorzugsweise zwischen 10 und 30 µm, besonders bevorzugt um 20 µm. Bei grösseren Schichtdicken kann die Wärme nicht schnell genug abgeführt werden, so dass sich mit steigender Dicke zunehmend kristalline Legierungsstrukturen herausbilden.

Die erhaltenen metallischen Gläser sind unporös. Ihre Oberfläche wird daher ausschliesslich durch ihre äussere geometrische Oberfläche gebildet. Diese Oberfläche beträgt bei der besonders bevorzugten Dicke der Metallbänder beziehungsweise Flakes von 20 µm nur etwa 0,5 m²/g. Dagegen weisen zum Beispiel aktivierte Nickelpulverkatalysatoren spezifische Metalloberflächen von mehr als 100 m²/g und auf Aktivkohle geträgerte feindisperse Palladiumkatalysatoren solche von mehr als 40 m²/g auf.

Wegen der geringen Metalloberfläche sind metallische Gläser bisher nicht als Katalysatoren für technische Prozesse verwendet worden. Ihre katalytischen Eigenschaften wurden jedoch intensiv untersucht. Sehr häufig wurden hohe Turnoverfrequenzen (Aktivitäten pro Reaktionszentrum und Zeit) gefunden. Einen Ueberblick über den Stand der Technik zur Katalyse an metallischen Gläsern gibt der Artikel "Glassy Metals in Catalysis" von A. Baiker in "Topics in Applied Physics" Vol. 72, Seiten 121-162, Ed. H. Beck and H.-J. Güntherodt, Springer Verlag 1994.

Es wurde unter anderem gefunden, dass metallische Gläser eine Vielzahl von Hydrierreaktionen katalysieren können, wie zum Beispiel die Hydrierung von Stickstoff, Ethen oder Butadien (Europäische Patentschrift 0 173 088 B1) bei Temperaturen im Bereich von 90°C bis 450°C und Gasdrücken von unter 10 bar.

Die katalytische Aktivität hängt von der jeweiligen Legierungszusammensetzung des metallischen Glases ab. Pd₈₁Si₁₉-Gläser erwiesen sich beispielsweise als besonders geeignet für die selektive Hydrierung von Alkinen zu Alkenen [A. Molnar, G.V. Smith and M. Bartok; J. Catal. **101**, 67-72 (1986)]. Diese Hydrierungen wurden bei Raumtemperatur und bei atmosphärischem Wasserstoffdruck durchgeführt.

Trotz der prinzipiellen Eignung metallischer Gläser für katalytische Hydrierungen wurden jedoch die geringe Metalloberfläche, die geringe thermische Stabilität und die Bildung von stabilen Oxidschichten auf der Oberfläche der metallischen Gläser während ihrer Herstellung als schwerwiegende Hindernisse für deren Verwendung in technischen Hydrierverfahren angesehen [D. Gasser und A. Baiker, Applied Catalysis, **48**, 279-294 (1989)]. Ueberdies war eine Versprödung der metallischen Gläser durch die Einwirkung des Wasserstoffs zu befürchten, die schliesslich zu deren Pulverisierung führen würde.

Aufgabe der vorliegenden Erfindung ist es, ein technisches Verfahren zur katalytischen Hydrierung organischer Verbindungen an metallischen Gläsern bereitzustellen , welches die aus dem Stand der Technik bekannten Hindernisse für den Einsatz metallischer Gläser bei technischen Hydrierprozessen überwindet und Hydrierungen mit hoher Raumzeitausbeute und guter Langzeitstabilität der katalytischen Aktivität ermöglicht.

Diese Aufgabe wird dadurch gelöst, dass die Hydrierung von organischen Verbindungen in Gegenwart metallischer Gläser (Katalysatoren aus amorphen Metall-Legierungen) und in einem Lösungsmittel so durchgeführt wird, dass die Hydrierung unter nahekritischen oder überkritischen Bedingungen des Lösungsmittels erfolgt [für die allgemein geläufigen Begriffe "nahekritisch" und "überkritisch" (near-critical bzw. supercritical) siehe beispielsweise den Artikel "Interaction of Density, Viscosity and Interfacial Tension in Countercurrent Extraction with Near-Critical Fluids" in "High Pressure Chemical Engineering", Seiten 191-197, Ed. Ph. Rudolf von Rohr and Ch. Trepp, Elsevier Science B.V. 1996, und die darin zitierten Literaturstellen]. Bei dem erfindungsgemässen Verfahren handelt es sich somit um ein Verfahren zur katalytischen Hydrierung einer organischen Verbindung an einem Katalysator aus einer amorphen Metall-Legierung und in einem Lösungsmittel; dieses Verfahren ist dadurch gekennzeichnet, dass man die Hydrierung unter nahekritischen oder überkritischen Bedingungen des Lösungsmittels und bei einem Wasserstoffpartialdruck zwischen 5 und 400 bar (0,5 und 40MPa) durchführt.

Die dabei angewendeten Reaktionstemperaturen liegen zweckmässigerweise im Bereich zwischen der Raumtemperatur und 300°C und ähneln somit denen, die auch bei den entsprechenden konventionellen Hydrierungen verwendet werden.

Es werden als Katalysatoren im erfindungsgemässen Verfahren metallische Gläser aus Legierungsmischungen mit eutektischem Schmelzpunkt eingesetzt. Die Eutektika sind aus den bekannten Phasendiagrammen der Legierierungsmischungen ersichtlich. Die amorphe Struktur der metallischen Gläser wird - wie bereits oben erwähnt - durch Schockkühlung der Legierungsschmelzen erreicht. Bevorzugt werden Legierungen von einem Metall aus der Gruppe Palladium, Eisen, Kupfer, Nickel und Vanadium und einem Metall aus der Gruppe Titan, Zirkon, Silizium, Germanium, Niob, Bor, Phosphor, Antimon und Wismut verwendet.

Es ist bekannt, dass metallische Gläser nur bei Legierungszusammensetzungen erhalten werden, die eutektischen Mischungen der Legierungskomponenten entsprechen oder diesen eutektischen Mischungen sehr nahe kommen. Typische Zusammensetzungen bevorzugter Legierungen, die im erfindungsgemässen Verfahren eingesetzt werden können, sind Pd₈₁Si₁₉, Fe₂₄Zr₇₆, Fe₉₁Zr₉, Ni₆₄Zr₃₆, Ni₆₄Ti₃₄ und Fe₈₅B₁₅. Ganz bevorzugt wird Pd₈₁Si₁₉ als amorphe Metall-Legierung verwendet. Die tatsächlichen Zusammensetzungen der verwendeten Legierungen sollten höchstens um 2 % (± 2 %) von diesen idealen Zusammensetzungen abweichen. Bei grösseren Abweichungen besteht die Gefahr, dass die metallischen Gläser einen zunehmenden Anteil an kristallinen Bereichen aufweisen.

Im Rahmen der vorliegenden Erfindung wurde gefunden, dass durch die Erhöhung des Wasserstoffpartialdruckes auf Werte über 5 bar (0,5 MPa) die katalytische Aktivität der metallischen Gläser so weit gesteigert werden kann, dass trotz der geringen metallischen Oberfläche Raumzeitausbeuten möglich werden, die gleich oder besser sind als die Werte, die mit konventionellen Trägerkatalysatoren mit wesentlich grösserer metallischer Oberfläche erreicht werden, wobei überraschenderweise die befürchtete Wasserstoffversprödung nicht auftritt. Wie bereits oben angedeutet, beträgt der Wasserstoffpartialdruck vorzugsweise nicht mehr als 400 bar (40MPa).

Für die Durchführung des erfindungsgemässen Verfahrens können die metallischen Gläser in Form von Bändern oder Flakes eingesetzt werden. Die Verwendung in Form von Flakes ist bevorzugt. Sowohl die Flakes als auch die Bänder haben zweckmässigerweise Schichtdicken im Bereich zwischen 5 und 50 µm, vorzugsweise zwischen 10 und 30 µm, besonders bevorzugt um 20 µm. Oberhalb von 50 µm Dicke werden wegen der ungenügend schnellen Wärmeabfuhr bei der Schockkühlung keine ausreichend amorphen Metall-Legierungen mehr erhalten. Bevorzugt werden Flakes mit mittleren Flächen von 0,5 bis 30 mm² verwendet. Solche Flakes können entweder schon bei der Herstellung der metallischen Gläser erzeugt oder erst nachträglich durch Mahlen aus Bändern erhalten werden.

Bei der Durchführung des erfindungsgemässen Verfahrens hängt die geeignete Auswahl des Lösungsmittels von dem zu hydrierenden Edukt ab. Verwendet werden können insbesondere aromatische und aliphatische Kohlenwasserstoffe, wie Benzol, Toluol, Propan oder Butan; Kohlendioxid; Alkohole, wie Methanol oder Ethanol; oder Mischungen davon.

Bevorzugt wird in diesem Verfahren als Lösungsmittel überkritisches Kohlendioxid [dessen kritische Temperatur etwa 31°C und kritischer Druck etwa 73 bar (7,3 MPa) beträgt], gegebenenfalls in Mischung mit Propan oder Butan, verwendet. Gemäss der Deutschen Patentpublikation (DOS) 44 05 029 A1 weist überkritisches Kohlendioxid in Hydrierreaktionen hervorragende Eigenschaften auf. Es besitzt sowohl für Wasserstoff als auch für viele organische Verbindungen ein gutes Lösevermögen. Durch seinen überkritischen Zustand besitzt es bei relativ hoher Dichte eine geringe Viskosität. Nach Durchlaufen des Reaktors kann es in der Regel in einfacher Weise ohne Umweltbelastungen durch Dekomprimieren vom Produkt abgetrennt und rezykliert werden.

Es können ansonsten aus konventionellen Verfahren bekannte Techniken eingesetzt werden. So können die Hydrierungen an einem Festbettreaktor durchgeführt werden, wobei der Wasserstoff im Gegenstrom oder Gleichstrom eingesetzt werden kann. Bevorzugt wird das Verfahren für kontinuierliche Hydrierungen eingesetzt.

Das erfindungsgemässe Verfahren kann auf alle gängigen Hydrierreaktionen an organischen Verbindungen angewendet werden. Es sind sowohl unselektive als auch selektive Hydrierreaktionen mit diesem Verfahren durchführbar. Beispiele sind die selektive oder vollständige Hydrierung der Doppelbindungen von Fettsäuren, die Hydrierung von Fettsäuren zu Fettalkoholen, die Zuckerhydrierung, die Hydrierung von Halogennitroaromaten zu Halogenaminen, die Ringhydrierung aromatischer Verbindungen und ganz allgemein die selektive Hydrierung von Alkinen zu Alkenen. Besonders bevorzugt wird das erfindungsgemässe Verfahren zur Hydrierung von 3,7,11,15-Tetramethyl-1-hexadecin-3-ol (Dehydroisophytol) zu 3,7,11,15-Tetramethyl-1-hexadecen-3-ol (Isophytol) verwendet.

Nicht alle metallischen Gläser sind für jede der aufgezählten Reaktionen gleich gut geeignet. Die katalytische Aktivität für eine bestimmte Reaktion hängt von der Zusammensetzung der gewählten Metall-Legierung ab. Einen Hinweis für die Auswahl der am besten geeigneten metallischen Gläser geben die bei den entsprechenden konventionellen Hydrierverfahren verwendeten Katalysatoren. So ist zum Beispiel das metallische Glas mit der Zusammensetzung Pd₈₁Si₁₉ sehr gut für alle Hydrierungen geeignet, bei denen gemäss dem Stand der Technik Palladiumkatalysatoren eingesetzt werden. Beispiele hierfür sind die selektiven Hydrierungen von Alkinen zu Alkenen. Wie auch von den konventionellen Katalysatoren bekannt, können die metallischen Gläser zur Erhöhung der Selektivität vergiftet werden. Hierzu werden häufig Blei- und Schwefelverbindungen, z.B. Bleiacetat bzw. 1,2-Bis-(2-hydroxyethylthio)ethan, allein oder in Kombination eingesetzt.

Bleiverbindungen eignen sich insbesondere zur Vergiftung von Palladium aufweisenden erfindungsgemäss verwendeten Katalysatoren bei der selektiven Hydrierung von Alkinen zu Alkenen und sind im allgemeinen zu diesem Zweck die bevorzugten vergiftenden Verbindungen. Die aufgenommene Bleimenge (Bleiatome, Pb) ist mit der Anzahl der Palladium-Oberflächen-Atome (Pd) korreliert: ein Atomverhältnis Pd:Pb von etwa 2:1 erweist sich als am geeignetesten. Bekanntlich kann die Bestimmung der Pd mit Kohlenmonoxid-Chemiesorption erfolgen. Als Alternative zu Bleiverbindungen können weitere Uebergangsmetalle aufweisende Verbindungen, wie beispielsweise Zink-, Zinn-, Mangan- und Kupferverbindungen, sowie Verbindungen mit Ligandfunktionen zu Palladium, wie beispielsweise Schwefelverbindungen, u.a. Sulfide, Thiole und Dimethylsulfoxid; Amine, z.B. Pyridin, γ-Collidin, Chinolin, Chinaldin und Piperidin; Phosphine; und Kohlenmonoxid, zur Erhöhung der Selektivität von Palladium für die Hydrierung von Alkinen zu Alkenen verwendet werden. Vergiftende Schwefelverbindungen eignen sich insbesondere bei der selektiven Hydrierung von Dehydroisophytol zu Isophytol gemäss der vorliegenden Erfindung. Es wird beispielsweise beim Einsatz von 1,2-Bis-(2-hydroxyethylthio)ethan zu diesem Zweck geeigneterweise etwa 0,005 bis etwa 0,1 Gewichtsprozent dieser Verbindung bezogen auf die Menge Edukt verwendet.

Die metallischen Gläser weisen im erfindungsgemässen Verfahren vielfältige Vorteile gegenüber der Verwendung von konventionellen Trägerkatalysatoren oder aktivierten Metallkatalysatoren auf. Die metallischen Gläser sind unporös, d.h. porenfrei. Dadurch entfallen an diesen Katalysatoren Probleme mit zu kleinen Diffusionsgeschwindigkeiten in den Poren konventioneller, poröser Katalysatorsysteme. Häufig ist die Diffusionsgeschwindigkeit viel kleiner als die Reaktionsgeschwindigkeit, was dazu führt, dass das bei der katalytischen Reaktion gebildete Produkt übermässig lange am Katalysator bleibt. Diese Tatsache ist für die Durchführung selektiver Reaktionen von Nachteil. Bei den porenfreien metallischen Gläsern tritt dieses Problem naturgemäss nicht auf.

Einen grossen Vorteil stellt auch die quasimetallische Wärmeleitfähigkeit der metallischen Gläser sowohl bei exothermen als auch bei endothemen Reaktionen dar. Im erfindungsgemässen Verfahren führt die Verwendung von zum Beispiel Pd₈₁Si₁₉ zu einer gleichmässigen Temperatur im Reaktorbett gegenüber konventionellen Palladium-Trägerkatalysatoren, bei denen aufgrund der schlechten Wärmeleitfähigkeit der im allgemeinen verwendeten oxidischen Trägermaterialien, z.B. Aluminium-, Siliziumund Titanoxid, Ueberhitzungen im Reaktor auftreten können. Zur Vermeidung solcher Ueberhitzungen muss zum Beispiel durch stärkere Verdünnung des Eduktes mit einem Lösungsmittel für eine bessere Wärmeabfuhr gesorgt werden.

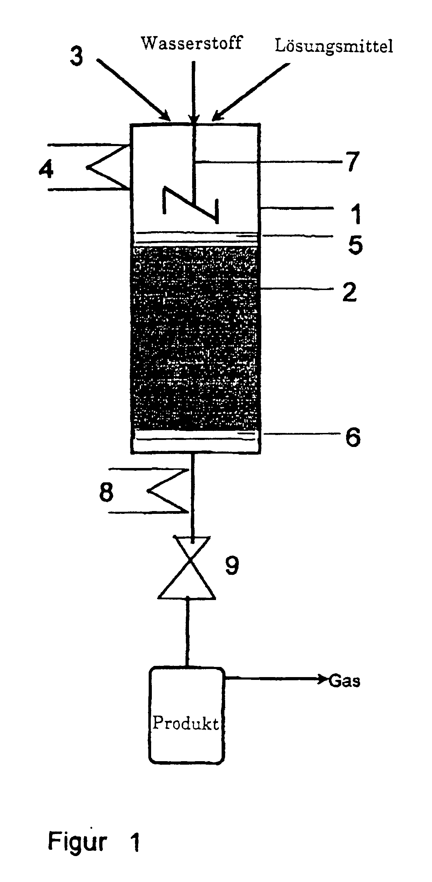

Die folgenden Beispiele veranschaulichen das erfindungsgemässe Verfahren. Figur 1 zeigt einen Rohrreaktor für die kontinuierliche Hydrierung von organischen Verbindungen.

### Beispiel 1

In dem in Figur 1 dargestellten Rohrreaktor wurde das Allen 6,10,14-Trimethyl-4,5-pentadecadien-2-on kontinuierlich zu 6,10,14-Trimethylpentadecan-2-on hydriert. Der Rohrreaktor bestand aus einem Edelstahlrohr 1, welches auf seiner Innenfläche mit einer 2,5 mm dicken Polytetrafluorethylen-Beschichtung versehen war. Der freie Innendurchmesser des Reaktors betrug 20 mm. Als Katalysator 2 wurden 74 g amorphes Pd₈₁Si₁₉ in Form von Flakes in den Reaktor gefüllt. Die Flakes wurden durch Mahlen von bandförmigem Material bei den Temperaturen des flüssigen Stickstoffs erhalten. Der mittlere Durchmesser der Flakes lag bei 5 mm, ihre Dicke bei 20 µm und ihre Schüttdichte bei 2,5 g/cm³.

Die Katalysatorschicht befand sich zwischen zwei Fritten 5 und 6. Im Kopf des Reaktors war ein Rührer 7 angeordnet.

Die Hydrierungen wurden bei einem Druck von 140 bar (14 MPa) unter Verwendung von Kohlendioxid (CO₂) als Lösungsmittel durchgeführt. Kohlendioxid lag unter den Bedingungen im Reaktor im überkritischen Zustand vor.

Edukt 3, Wasserstoff und Kohlendioxid wurden am Kopf des Reaktors aufgegeben und über die Katalysatorschüttung geleitet. Das Reaktionsgemisch wurde oberhalb der Katalysatorschüttung durch einen Heizmantel 4 auf die notwendige Reaktionstemperatur von 140° bis 200°C erwärmt. Nach Durchlaufen der Katalysatorschüttung wurde das Reaktionsgemisch in einem Kühler 8 abgekühlt, und das Produkt durch Dekompression 9 vom Lösungsmittel Kohlendioxid und vom unverbrauchten Wasserstoff abgetrennt.

Das Produktgemisch wurde gaschromatisch (GC) analysiert. Tabelle 1 gibt die Ergebnisse dieser Messungen für eine kontinuierliche Hydrierung über die Dauer von 1630 Minuten wieder. Dabei wurden 2,04 Mol Edukt und 21,42 Mol Wasserstoff pro Stunde dem Reaktor zugeführt. Die Raumzeitausbeute des Produktes 6,10,14-Trimethyl-pentadecan-2-on betrug 123 Mol pro Liter pro Stunde (Mol/lh).

**Tabelle 1**

| Zeit [min] | CO₂ [g/h] | Temperatur [° C] | Produkt [GC %] |
|---|---|---|---|
| 0 | 1200 | 192 | 88,9 |
| 50 | 1200 | 192 | 88,9 |
| 140 | 1200 | 192 | 89,2 |
| 225 | 1200 | 192 | 88,9 |
| 280 | 1200 | 192 | 89,0 |
| 340 | 1200 | 181 | 88,0 |
| 395 | 1200 | 181 | 88,1 |
| 445 | 1200 | 181 | 86,8 |
| 480 | 900 | 166 | 87,5 |
| 520 | 900 | 166 | 86,0 |
| 565 | 900 | 166 | 86,3 |
| 645 | 900 | 166 | 86,6 |
| 765 | 600 | 166 | 86,1 |
| 885 | 300 | 160 | 88,2 |
| 1005 | 0 | 154 | 88,3 |
| 1125 | 300 | 159 | 87,9 |
| 1245 | 600 | 162 | 85,9 |
| 1365 | 900 | 165 | 84,9 |
| 1505 | 600 | 163 | 87,2 |
| 1545 | 0 | 142 | 88,6 |
| 1585 | 0 | 142 | 88,0 |
| 1630 | 0 | 142 | 89,0 |

### Beispiel 2

Der Reaktor von Beispiel 1 wurde verwendet, um das Alkin 3,7,11,15-Tetramethyl-1-hexadecin-3-ol (Dehydroisophytol) zum Alken 3,7,11,15-Tetramethyl-1-hexadecen-3-ol (Isophytol) zu hydrieren. Der Reaktor wurde mit 80 g gemahlenem Pd₈₁Si₁₉ gefüllt. Als Lösungsmittel wurden 1080 g CO₂ pro Stunde zudosiert. Der Reaktionsdruck betrug 140 bar (14 MPa).

Tabelle 2 gibt die gaschromatischen Analysen des Produktionsgemisches wieder sowie die jeweilige Raumzeitausbeute für das ebenfalls gebildete Alkan 3,7,11,15-Tetramethyl-hexadecan-3-ol.

**Tabelle 2:**

| Hydrierbedingungen | | | | Analyse des Produktgemisches | | | |
|---|---|---|---|---|---|---|---|
| Zeit | H₂ | Alkin | Temp. | Alkin | Alken | Alkan | |
| min | mol/h | mol/h | °C | GC% | GC% | GC% | RZA |
| 67 | 1,8 | 0,36 | 129 | 0,0 | 0,5 | 96,9 | 22 |
| 102 | 1,8 | 0,36 | 129 | 0,0 | 0,4 | 96,8 | 22 |
| 189 | 2,7 | 0,54 | 135 | 0,0 | 0,4 | 96,6 | 33 |
| 202 | 2,7 | 0,54 | 135 | 0,0 | 0,4 | 96,5 | 33 |
| 283 | 3,6 | 0,72 | 141 | 0,0 | 0,3 | 95,9 | 44 |
| 292 | 3,6 | 0,72 | 141 | 0,0 | 0,4 | 96,3 | 44 |
| 361 | 4,5 | 0,90 | 147 | 0,0 | 0,5 | 95,5 | 54 |
| 375 | 4,5 | 0,90 | 147 | 0,0 | 0,3 | 95,1 | 54 |
| | | | | | | | |
| 89 | 2,3 | 0,90 | 149 | 0,0 | 0,4 | 95,5 | |
| 109 | 2,3 | 0,90 | 149 | 0,0 | 0,4 | 95,8 | |
| 195 | 1,5 | 0,90 | 149 | 1,3 | 20,8 | 74,7 | |
| 207 | 1,5 | 0,90 | 149 | 1,5 | 22,9 | 72,4 | |
| 265 | 1,1 | 0,90 | 143 | 5,4 | 27,8 | 63,8 | |
| 274 | 1,1 | 0,90 | 143 | 9,8 | 32,9 | 54,5 | |
| 318 | 1,1 | 1,26 | 144 | 22,6 | 33,9 | 41,0 | |
| 327 | 1,1 | 1,26 | 144 | 26,4 | 34,1 | 37,0 | |
| 392 | 1,1 | 1,62 | 145 | 27,2 | 33,5 | 36,9 | |
| 398 | 1,1 | 1,62 | 145 | 27,0 | 33,4 | 37,1 | |
| 430 | 1,1 | 0,90 | 131 | 11,5 | 29,3 | 56,8 | |
| 527 | 1,1 | 0,90 | 131 | 11,3 | 29,9 | 56,2 | |
| RZA: Raumzeitausbeute in Mol/lh | | | | | | | |

### Beispiel 3

Dehydroisophytol wurde im bisher beschriebenen Rohrreaktor (Figur 1) selektiv zu Isophytol hydriert. Hierzu wurde der Pd₈₁Si₁₉-Katalysator wie folgt mit einer Bleiverbindung vergiftet. Das gemahlene, metallische Glas wurde für die Dauer von 3 Stunden bei Raumtemperatur mit einer übersättigten Lösung von Bleiacetat in Ethanol überschichtet. Nach Abgiessen der Lösung wurden die Flakes im Vakuum getrocknet, danach für die Dauer von 3 Stunden unter einer Atmosphäre aus Wasserstoffgas gelagert und anschliessend mit entsalzenem Wasser und Aceton gewaschen.

Für die Hydrierungen wurden 48,3 g der so behandelten Flakes in den Reaktor eingefüllt. Die Reaktionsbedingungen und Analysenergebnisse des Produktgemisches gibt Tabelle 3 wieder.

**Tabelle 3**

| Hydrierbedingungen | | | | | Analyse des Produktgemisches | | | |
|---|---|---|---|---|---|---|---|---|
| Zeit | CO₂ | H₂ | Edukt | Temp. | Edukt | Alken | | Alkan |
| min | g/h | mol/h | mol/h | °C | GC% | GC% | RZA | GC % |
| 80 | 1200 | 3,2 | 1,6 | 84 | 0,0 | 87,9 | 75 | 9,8 |
| 195 | 1200 | 3,0 | 1,6 | 84 | 0,0 | 90,2 | 75 | 6,7 |
| 330 | 1200 | 2,8 | 1,6 | 84 | 0,0 | 90,9 | 75 | 6,2 |
| 430 | 1200 | 2,7 | 1,6 | 84 | 0,0 | 90,8 | 75 | 6,1 |
| 480 | 1200 | 2,5 | 1,6 | 83 | 0,0 | 91,1 | 75 | 6,2 |
| | | | | | | | | |
| 95 | 1200 | 3,2 | 1,6 | 79 | 0,2 | 90,9 | 75 | 6,7 |
| 220 | 1200 | 3,0 | 1,6 | 78 | 0,2 | 91,2 | 75 | 6,5 |
| 360 | 1200 | 2,8 | 1,6 | 78 | 0,5 | 91,0 | 75 | 5,8 |
| 430 | 1200 | 2,7 | 1,6 | 77 | 0,8 | 90,9 | 75 | 5,9 |
| 480 | 1200 | 2,5 | 1,6 | 74 | 4,2 | 88,6 | 75 | 5,3 |
| | | | | | | | | |
| 90 | 840 | 2,8 | 1,6 | 97 | 0,5 | 87,8 | 75 | 9,5 |
| 200 | 840 | 2,7 | 1,6 | 97 | 0,0 | 89,5 | 75 | 8,2 |
| 305 | 840 | 2,5 | 1,6 | 96 | 0,0 | 90,3 | 75 | 8,6 |
| 400 | 840 | 2,4 | 1,6 | 97 | 0,0 | 90,7 | 75 | 7,0 |
| 470 | 840 | 2,3 | 1,6 | 96 | 0,4 | 91,1 | 75 | 7,1 |
| | | | | | | | | |
| 235 | 840 | 2,7 | 1,6 | 95 | 0,7 | 89,3 | 75 | 8,1 |
| 325 | 840 | 2,7 | 1,5 | 91 | 0,3 | 90,5 | 70 | 7,2 |
| 415 | 840 | 2,7 | 1,4 | 88 | 0,1 | 91,1 | 65 | 6,8 |
| 475 | 840 | 2,7 | 1,3 | 84 | 0,1 | 90,3 | 60 | 7,6 |
| RZA: Raumzeitausbeute in Mol/lh | | | | | | | | |

### Beispiel 4

Zur weiteren Erhöhung der Selektivität der Hydrierung von Beispiel 3 wurde als Katalysator wieder die mit Blei vergiftete Pd₈₁Si₁₉-Legierung verwendet. Zusätzlich wurde der Katalysator mit einer Schwefelverbindung vergiftet. Hierzu wurde 1,2-Bis-(2-hydroxyethylthio)ethan dem Eduktgemisch zudosiert. Die genauen Versuchsbedingungen und Ergebnisse sind in Tabelle 4 aufgeführt; es handelt sich dabei um den Vergleich einer Hydrierung an einem Palladium-Trägerkatalysator.

### Vergleichsbeispiel

Analog zu Beispiel 4 wurde Dehydroisophytol an einem konventionellen Palladium-Trägerkatalysator zu Isophytol hydriert. Der Katalysator enthielt 4 Gew.-% Palladium sowie 4 Gew.-% Blei auf einem Träger aus einem organofunktionellen Polysiloxan. Dieser Katalysator ist in der Deutschen Patentschrift 41 10 706 beschrieben. Die Versuchsbedingungen und Ergebnisse sind der Tabelle 4 zu entnehmen.

**Tabelle 4:**

| Kontinuierliche Hydrierung von Dehydroisophytol zu Isophytol | | |
|---|---|---|
| | Beispiel 4 | Vergleichsbeispiel |
| Katalysator | Pd₈₁Si₁₉ | 4 Gew.-% Pd + |
| | | 4 Gew.-% Pb |
| Katalysatormenge [g] | 44 | 34 |
| Katalysatorvolumen [cm³] | 20 | 120 |
| Pd-Nutzfläche [m²] | 0,5 | 40 |
| Eduktdosierung [g/h] + 1Gew. Schwefelverb. | 460 | 430 |
| CO₂ [g/h] | 660 | 1980 |
| H₂-Druck [bar] | 140 | 140 |
| Temperatur [°C] | 110 | 110 |
| Ausbeute [%] | 97 | 97 |
| Raumzeitausbeute [kg/lh] | 23 | 4 |

## Patentansprüche

1. Verfahren zur katalytischen Hydrierung einer organischen Verbindung an einem Katalysator aus einer amorphen Metall-Legierung und in einem Lösungsmittel, **dadurch gekennzeichnet, dass** man die Hydrierung unter nahekritischen oder überkritischen Bedingungen des Lösungsmittels und bei einem Wasserstoffpartialdruck zwischen 5 und 400 bar (0,5 und 40 MPa) durchführt.

2. Verfahren gemäss Anspruch 1, **dadurch gekennzeichnet, dass** man als amorphe Metall-Legierung eine Legierung von einem Metall aus der Gruppe Palladium, Eisen, Kupfer, Nickel und Vanadium und einem Metall aus der Gruppe Titan, Zirkon, Silizium, Germanium, Niob, Bor, Phosphor, Antimon und Wismut verwendet.

3. Verfahren gemäss Anspruch 2, **dadurch gekennzeichnet, dass** es sich bei der amorphen Metall-Legierung um Pd₈₁Si₁₉, Fe₂₄Zr₇₆, Fe₉₁Zr₉, Ni₆₄Zr₃₆, Ni₆₄Ti₃₄ oder Fe₈₅B₁₅, vorzugsweise um Pd₈₁Si₁₉, handelt.

4. Verfahren gemäss einem der Ansprüche 1-3, **dadurch gekennzeichnet, dass** die amorphe Metall-Legierung in Form von Bändern oder Flakes mit einer Schichtdicke im Bereich zwischen 5 und 50 µm, vorzugsweise zwischen 10 und 30 µm, besonders bevorzugt um 20 µm, eingesetzt wird.

5. Verfahren gemäss einem der Ansprüche 1-4, **dadurch gekennzeichnet, dass** die amorphe Metall-Legierung in Form von Flakes mit einer mittleren Fläche von 0,5 bis 30 mm² eingesetzt wird.

6. Verfahren gemäss einem der Ansprüche 1-5, **dadurch gekennzeichnet, dass** die amorphe Metall-Legierung mit einer Blei- oder Schwefelverbindung oder einer Kombination davon vergiftet ist.

7. Verfahren gemäss einem der Ansprüche 1-6, **dadurch gekennzeichnet, dass** als Lösungsmittel ein aromatischer oder aliphatischer Kohlenwasserstoff, wie Benzol, Toluol, Propan oder Butan; Kohlendioxid; ein Alkohol, wie Methanol oder Ethanol; oder eine Mischung davon, vorzugsweise überkritisches Kohlendioxid, verwendet wird.

8. Verfahren gemäss einem der Ansprüche 1-7, **dadurch gekennzeichnet, dass** man die Hydrierung kontinuierlich durchführt.

9. Verfahren gemäss einem der Ansprüche 1-8 **dadurch gekennzeichnet, dass** man 3,7,11,15-Tetramethyl-1-hexadecin-3-ol (Dehydroisophytol) zu 3,7,11,15-Tetramethyl-1-hexadecen-3-ol (Isophytol) hydriert.

## Claims

1. A process for the catalytic hydrogenation of an organic compound on a catalyst of an amorphous metal alloy and in a solvent, which process comprises carrying out the hydrogenation under near-critical or supercritical conditions of the solvent and at a hydrogen partial pressure between 5 and 400 bar (0.5 and 40 MPa).

2. A process according to claim 1, wherein an alloy of a metal from the group of palladium, iron, copper, nickel and vanadium and a metal from the group of titanium, zirconium, silicon, germanium, niobium, boron, phosphorus, antimony and bismuth is used as the amorphous metal alloy.

3. A process according to claim 2, wherein the amorphous metal alloy is Pd₈₁Si₁₉, Fe₂₄Zr₇₆, Fe₉₁Zr₉, Ni₆₄Zr₃₆, Ni₆₄Ti₃₄ or Fe₈₅B₁₅, preferably Pd₈₁Si₁₉.

4. A process according to any one of claims 1-3, wherein the amorphous metal alloy is used in the form of ribbons or flakes having a layer thickness in the range between 5 and 50 µm, preferably between 10 and 30 µm, especially about 20 µm.

5. A process according to any one of claims 1-4, wherein the amorphous metal alloy is used in the form of flakes having an average area of 0.5 to 30 mm².

6. A process according to any one of claims 1-5, wherein the amorphous metal alloy is poisoned with a lead or sulphur compound or a combination thereof.

7. A process according to any one of claims 1-6, wherein an aromatic and aliphatic hydrocarbon, such as benzene, toluene, propane or butane; carbon dioxide; an alcohol, such as methanol or ethanol; or a mixture thereof, preferably supercritical carbon dioxide, is used as the solvent.

8. A process according to any one of claims 1-7, wherein the hydrogenation is carried out continuously.

9. A process according to any one of claims 1-8, wherein 3,7,11,15-tetramethyl-1-hexadecyn-3-ol (dehydroisophytol) is hydrogenated to 3,7,11,15-tetramethyl-1-hexadecen-3-ol (isophytol).

## Revendications

1. Procédé pour l'hydrogénation catalytique d'un composé organique sur un catalyseur à partir d'un alliage métallique amorphe et dans un solvant, **caractérisé en ce que** l'hydrogénation est réalisée à l'état quasi critique ou surcritique du solvant et avec une pression partielle d'hydrogéne située entre 5 et 400 bar (entre 0,5 et 40 MPa).

2. Procédé selon la revendication 1, **caractérisé en ce que** l'alliage métallique amorphe utilisé est constitué d'un alliage à base d'un métal du groupe palladium, fer, cuivre, nickel et vanadin et d'un métal du groupe titan, zircon, silice, germanium, niobium, bore, phosphore, antimoine et bismuth.

3. Procédé selon la revendication 2, **caractérisé en ce que** qu'il s'agit pour l'alliage métallique amorphe de Pd₈₁Si₁₉, Fe₂₄Zr₇₆, Fe₉₁Zr₉, Ni₆₄Zr₃₆, Ni₆₄Ti₃₄ ou Fe₈₅B₁₅, de préférence de Pd₈₁Si₁₉.

4. Procédé selon l'une des revendications 1-3, **caractérisé en ce que** l'alliage métallique amorphe est mis en oeuvre sous forme de bandes ou lamelles d'une épaisseur de couche située dans une plage entre 5 et 50 µm, de préférence entre 10 et 30 µm, avec une préférence particulière autour de 20 µm.

5. Procédé selon l'une des revendications 1-4, **caractérisé en ce que** l'alliage métallique amorphe est mis en oeuvre sous forme de lamelles d'une surface moyenne entre 0,5 et 30 mm².

6. Procédé selon l'une des revendications 1-5, **caractérisé en ce que** l'alliage métallique amorphe est contaminé par un composé de plomb ou de soufre ou d'une combinaison des deux.

7. Procédé selon l'une des revendications 1-6, **caractérisé en ce que** le solvant utilisé est un hydrocarbure aromatique ou aliphatique comme le benzène, le toluène, le propane ou le butane ; du dioxyde de carbone ; un alcool comme le méthanol ou l'éthanol ; ou un mélange de ceux-ci, de préférence du dioxyde de carbone surcritique.

8. Procédé selon l'une des revendications 1-7, **caractérisé en ce que** l'hydrogénation est réalisée de manière continue.

9. Procédé selon l'une des revendications 1-8, **caractérisé en ce que** l'on transforme par hydrogénation du 3, 7, 11, 15-tétraméthylhexadéc-1-yn-3-ol (déshydroisophytol) en 3, 7, 11, 15-tétraméthylhexadéc-1-én-3-ol (isophytol).
